# EUROPÄISCHE PATENTSCHRIFT

(11) **EP 2 234 575 B1**
(45) Veröffentlichungstag und Bekanntmachung des Hinweises auf die Patenterteilung: **05.10.2016**
(21) Anmeldenummer: 08862676.7
(22) Anmeldetag: 27.10.2008
(51) Int. Cl.: A61K 8/11, A61K 8/81, A61Q 5/08

(54) **MITTEL ZUM AUFHELLEN VON KERATINHALTIGEN FASERN MIT BESCHICHTETEN PARTIKELN**
MEANS FOR BRIGHTENING KERATIN-CONTAINING FIBERS COMPRISING COATED PARTICLES
AGENTS DE DÉCOLORATION DE FIBRES KÉRATINIQUES CONTENANT DES PARTICULES REVÊTUES

(30) Priorität: 18.12.2007 DE 102007061508; 03.04.2008 DE 102008017434
(43) Veröffentlichungstag der Anmeldung: 06.10.2010
(73) Patentinhaber: Henkel AG & Co. KGaA, 40589 Düsseldorf (DE)
(72) Erfinder: MANNECK, Hartmut, 23860 Klein Wesenberg (DE); KLEEN, Astrid, 20457 Hamburg (DE); AKRAM, Mustafa, 22455 Hamburg (DE); WELZ, Carolin, 22459 Hamburg (DE)
(86) Internationale Anmeldenummer: PCT/EP2008/064526
(87) Internationale Veröffentlichungsnummer: WO 2009/077252

(56) Entgegenhaltungen:
- EP-A1- 1 752 191
- DE-A1- 3 801 606
- DE-A1- 19 543 989
- DE-U1-202004 012 607

## Beschreibung

Die Erfindung betrifft Mittel zum Aufhellen von keratinhaltigen Fasern, insbesondere Blondiermittel für menschliche Haare.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die so genannten Blondierverfahren, im Wesentlichen nur dunkelblonde oder hellere Haare. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

So werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im Weiteren als "Blondiermittel" bzw. "Blondierpulver" bezeichnet. Alle aufgeführten Mengenangaben beziehen sich, soweit nicht anders ausgeführt, ausschließlich auf diese Zubereitungen.

Die vorliegende Erfindung bezieht sich auch auf Mittel zum Aufhellen von keratinhaltigen Fasern, insbesondere menschlichen Haaren.

In Blondiermitteln besteht das Problem, dass ein Oxidationsmittel, das dem Mittel zugesetzt wird, um ein Anwendungsprodukt zu erhalten, schnell zersetzt wird. Diese Zersetzungsreaktion wird insbesondere durch Alkalisierungsmittel beschleunigt. Bei Zersetzung von Wasserstoffperoxid tritt eine Gasentwicklung ein. Diese kann während der Handhabung der Blondiermittel je nach Intensität ein Gefahrenpotenzial für den Anwender in sich bergen.

Zudem werden Blondiermittel oft dazu benutzt, nur einzelne Haarsträhnen aufzuhellen, um eine optisch interessante Haarfärbung zu erzeugen. Beim herkömmlichen "Strähnchenprozess" werden einzelnen Haarsträhnen in Folien eingelegt, mit der Blondiermittelzubereitung beaufschlagt und in die Folie eingeschlagen. Da der Applikationsprozess langwierig ist, sind die Einwirkzeiten auf die nacheinander beaufschlagten Strähnen unterschiedlich, was zu unerwünschten weil unterschiedlichen Färbeergebnissen mit verschieden hellen Strähnen führt. Daher ist eine größere zeitliche Unabhängigkeit für den Anwender, insbesondere für den professionellen Anwender, beim Auftragen und Ausspülen der Blondiermittel wünschenswert.

Der vorliegenden Erfindung lag die Aufgabe zugrunde, Mittel zum Aufhellen von Keratinfasern bereitzustellen, die es ermöglichen, auch bei der "Strähnchentechnik" ein einheitliches Färbeergebnis zu erzielen. Das Überbleichen einzelner Strähnen, die im Prozess vor anderen Strähnen mit der Applikationsmischung beaufschlagt werden, sollte vermieden werden.

Es wurde nun gefunden, dass sich die vorstehenden Aufgaben lösen lassen, indem Blondiermittel bereitgestellt werden, in denen mindestens ein partikelförmiger Bestandteil enthalten ist, welcher eine Beschichtung aus bestimmten Stoffen aufweist. Besonders bevorzugte Ausführungsformen der vorliegenden Erfindung sehen vor, dass das Blondiermittel vollständig aus partikelförmigen Bestandteilen besteht, welche alle beschichtet sind.

Durch die Beschichtung des Blondiermittels wird der Start des eigentlichen Blondiervorgangs verzögert, und somit eine größere zeitliche Unabhängigkeit beim Auftragen der Blondierung für den Anwender erreicht. Auch im Falle einer schlechten Zeiteinhaltung seitens des Anwenders beim Ausspülen der Strähnen wird daher eine gleichmäßige und damit schonende Blondierung erhalten.

In einer ersten Ausführungsform betrifft die vorliegende Erfindung Mittel zum Aufhellen von Keratinfasern, insbesondere menschlichen Haaren, enthaltend mindestens einen partikelförmigen Bestandteil, der einen Partikelkern, enthaltend mindestens ein festes Alkalisierungsmittel ausgewählt aus Alkalimetall-carbonaten, -silikaten und -phosphaten umfasst und eine diesen Kern umgebende Hülle aufweist, dadurch gekennzeichnet, dass die Hülle zu mindestens 50 Gew.-% ihres Gewichts aus
a) Homo- und/oder Copolymeren von Methacrylsäure und/oder Methacrylsäureestern und/oder
b) Homo- und/oder Copolymeren von Vinylacetat
besteht.

In einer bevorzugten Ausführungsform besteht die den Partikelkern umgebende Hülle zu mindestens 70 Gew.-%, weiter bevorzugt zu mindestens 90 Gew.-% und insbesondere zu 100 Gew.-% ihres Gewichts aus
c) Homo- und/oder Copolymeren von Methacrylsäure und/oder Methacrylsäureestern und/oder
d) Homo- und/oder Copolymeren von Vinylacetat.

Nachfolgend werden die erfindungsgemäßen Mittel zum Aufhellen von Keratinfasern auch als "Blondiermittel", als "Aufhellmittel" bezeichnet. Diese Begriffe sind dabei nicht als beschränkend zu verstehen.

Erfindungsgemäße Mittel enthalten mindestens einen partikelförmigen Kern, welcher mit einer Hülle umgeben ist.

Erfindungsgemäße Mittel können in einer Vielzahl von Angebotsformen bereitgestellt werden, beispielsweise in Form von Pasten, Pulvern, Tabletten usw., solange sie mindestens einen partikelförmigen Inhaltsstoff enthalten, welcher mit einer Hülle umgeben ist. Besonders bevorzugt liegen die erfindungsgemäßen Mittel im Hinblick auf die Anwendungskonvenienz jedoch als Blondierpulver vor. Erfindungsgemäße Mittel, die als Partikelgemisch ("in Pulverform") vorliegen, sind bevorzugte Ausführungsformen der vorliegenden Erfindung.

In weiter bevorzugten Mitteln wird das Beschichtungsmaterial in einer bestimmten Menge auf die zu beschichtenden Partikel aufgebracht. Hier sind erfindungsgemäße Mittel bevorzugt, bei denen die Hülle 10 bis 70 Gew.-% des Gewichts der beschichteten Partikel ausmacht.

Eine bevorzugte Ausführungsform der vorliegenden Erfindung sieht Mittel vor, die als Partikelkern Peroxoverbindungen enthalten.

Erfindungsgemäß besonders geeignete Beschichtungsmittel sind Homo- und/oder Copolymere von Vinylacetat. Unter den Cpolymeren von Vinylacetat sind insbesondere dessen Copolymere mit Ethylen (Ethylen-Vinylacetat-Copolymere, Kurzzeichen EVA oder EVAC) und/oder mit Vinylchlorid [Kurzzeichen VCEVAC (oft auch fälschlich VCEVA) bzw. VCVAC (auch PVCA)] von Bedeutung und können erfindungsgemäß eingesetzt werden.

Besonders bevorzugt sind jedoch erfindungsgemäße Mittel, die das Hompolymer als Beschichtungsmaterial für die Partikel beinhalten, namentlich Mittel, bei denen die Hülle Polyvinylacetat enthält.

Polyvinylacetat (Kurzzeichen PVAC) wird durch radikalische Polymerisation von Essigsäurevinylester erhalten. Die Verknüpfung der Monomeren beim Aufbau der Polymerkette erfolgt in hohen Anteilen (bis zu 98%) als Kopf/Schwanz-Polymerisation und nur in geringem Maß als Kopf/Kopf-Polymerisation; die Makromoleküle der Polyvinylacetate enthalten also hauptsächlich Gruppierungen des Typs I (Kopf/Schwanz) und nur wenige des Typs II (Kopf/Kopf) als charakteristische Grundeinheiten:

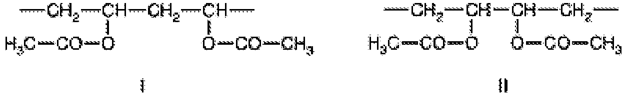

Die Herstellung der Polyvinylacetate kann nach Verfahren der Substanz-, Lösungs-, Suspensions- (Perl-) oder als Emulsionspolymerisation erfolgen. Bevorzugte Polyvinylacetate haben Molmassen von 10.000 - 1.500.000 g/mol und eine Molmassen-abhängige Glasübergangstemperatur von ca. 28°C. Sie sind amorphe, geruch- und geschmacklose Produkte mit hoher Licht- und Witterungs-Beständigkeit, unlöslich in Wasser und löslich in vielen organischen Lösungsmitteln (Ester, Ether, Ketone, halogenierte Kohlenwasserstoffe u.a.).

Zusätzlich zu Polyvinylacetat und/oder an dessen Stelle kann die Hülle auch bestimmte Homo- und/oder Copolymere von Methacrylsäure und/oder Methacrylsäureestern enthalten. Unter diesen sind die Copolymere von Methacrylsäure und/oder Methacrylsäureestern bevorzugt. Besonders bevorzugte Vertreter werden nachstehend beschrieben.

Ein besonders geeignetes Hüllmaterial stellen Copolymere aus Methacrylsäure, Methylacrylat und Methylmethacrylat dar. Diese enthalten Monomereinheiten der folgenden Strukturen: 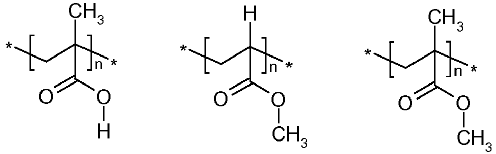 wobei die Indices n je nach Molmasse des Polymers variieren und nicht bedeuten, dass es sich um Blockcopolymere handelt. Vielmehr können Struktureinheiten im Molekül statistisch verteilt vorliegen.

In erfindungsgemäß bevorzugten Mitteln enthält die Hülle mindestens ein Copolymer aus Methacrylsäure, Methylacrylat und Methylmethacrylat.

In besonders bevorzugten Polymeren dieses Typs ist das molare Verhältnis von Methacrylsäure zu den beiden Estern > 1, vorzugsweise > 5. Besonders bevorzugte erfindungsgemäße Mittel sind dadurch gekennzeichnet, dass das molare Verhältnis von Estergruppen zu freien Carboxygruppen im Copolymer 1:5 bis 1:12 beträgt.

Noch weiter bevorzugte Polymere dieses Typs weisen Molmassen um 220 kDa auf. Besonders bevorzugte erfindungsgemäße Mittel sind dadurch gekennzeichnet, dass das Copolymer aus Methacrylsäure, Methylacrylat und Methylmethacrylat eine Molmasse von 200 bis 250 kDa aufweist.

Ein weiteres besonders geeignetes Hüllmaterial stellen Copolymere aus Methacrylsäure, und Ethylacrylat dar. Diese enthalten Monomereinheiten der folgenden Strukturen: 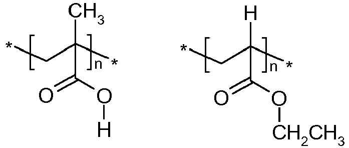 wobei die Indices n je nach Molmasse des Polymers variieren und nicht bedeuten, dass es sich um Blockcopolymere handelt. Vielmehr können Struktureinheiten im Molekül statistisch verteilt vorliegen.

In erfindungsgemäß bevorzugten Mitteln enthält die Hülle mindestens ein Copolymer aus Methacrylsäure und Ethylacrylat.

In besonders bevorzugten Polymeren dieses Typs ist das molare Verhältnis von Methacrylsäure zu dem Ester ca. 1. Besonders bevorzugte erfindungsgemäße Mittel sind dadurch gekennzeichnet, dass das molare Verhältnis von Estergruppen zu freien Carboxygruppen im Copolymer 1:0,9 bis 0,9:1 beträgt.

Noch weiter bevorzugte Polymere dieses Typs weisen Molmassen um 250 kDa auf. Besonders bevorzugte erfindungsgemäße Mittel sind dadurch gekennzeichnet, dass das Copolymer aus Methacrylsäure und Ethylacrylat eine Molmasse von 225 bis 275 kDa aufweist.

Ein weiteres besonders geeignetes Hüllmaterial stellen Copolymere dar, welche Methacrylsäureester mit kationischen Gruppen enthalten, insbesondere aus (ω-Trialkylammonioalkyl) methacrylate. Besonders bevorzugt sind erfindungsgemäße Mittel, bei denen die Hülle mindestens ein Copolymer aus (2-Trimethylammonioethyl) methacrylat chlorid enthält. Dieses enthält Monomereinheiten der Formel (I)

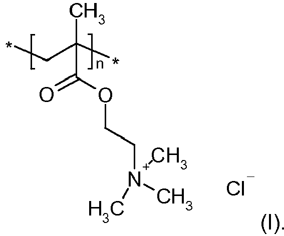

Als zusätzliche(s) Monomer(e) in Copolymeren mit Monomereinheiten der Formel (I) haben sich insbesondere Ester der Methacrylsäure und/oder Ester der Acrylsäure bewährt.

In bevorzugten Mittel enthält die Hülle der Partikel daher Copolymere aus (2-Trimethylammonioethyl) methacrylat chlorid und mindestens einem zusätzlichen Monomer, welches ausgewählt wird aus der Gruppe, die gebildet wird aus Ethylmethacrylat, Methylmethacrylat, Ethylacrylat und Methylacrylat.

Bevorzugt sind erfindungsgemäße Mittel, bei denen das Copolymer mit Monomereinheiten der Formel (I) als zusätzliche(s) Monomer(e) Ethylmethacrylat enthält.

Weiter bevorzugt sind erfindungsgemäße Mittel, bei denen das Copolymer mit Monomereinheiten der Formel (I) als zusätzliche(s) Monomer(e) Methylmethacrylat enthält.

Zusätzlich zu den weiteren Methacrylsäureestern oder an deren Stelle können auch Acrylsäureester in das Copolymer mit Monomereinheiten der Formel (I) einpolymerisiert werden. Hier sind erfindungsgemäße Mittel bevorzugt, bei denen das Copolymer mit Monomereinheiten der Formel (I) als zusätzliche(s) Monomer(e) Ethylacrylat enthält.

Weiter bevorzugt sind erfindungsgemäße Mittel, bei denen das Copolymer mit Monomereinheiten der Formel (I) als zusätzliche(s) Monomer(e) Methylacrylat enthält.

Ein weiteres besonders geeignetes Hüllmaterial stellen Copolymere aus Methylmethacrylat und Ethylacrylat dar. Diese enthalten Monomereinheiten der folgenden Strukturen: 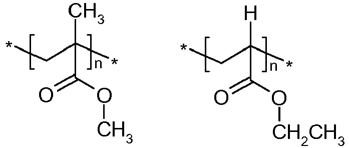 wobei die Indices n je nach Molmasse des Polymers variieren und nicht bedeuten, dass es sich um Blockcopolymere handelt. Vielmehr können Struktureinheiten im Molekül statistisch verteilt vorliegen.

In erfindungsgemäß bevorzugten Mitteln enthält die Hülle mindestens ein Copolymer aus Methylmethacrylat und Ethylacrylat.

In besonders bevorzugten Polymeren dieses Typs ist das molare Verhältnis von Methacrylsäure zu dem Ester ca. 1. Besonders bevorzugte erfindungsgemäße Mittel sind dadurch gekennzeichnet, dass das molare Verhältnis von Estergruppen zu freien Carboxygruppen im Copolymer 1:0,9 bis 0,9:1 beträgt.

Noch weiter bevorzugte Polymere dieses Typs weisen Molmassen um 800 kDa auf. Besonders bevorzugte erfindungsgemäße Mittel sind dadurch gekennzeichnet, dass das Copolymer aus Methylmethacrylat und Ethylacrylat eine Molmasse von 750 bis 850 kDa aufweist.

Erfindungsgemäß besteht die Hülle zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Homo- und/oder Copolymeren von Methacrylsäure und/oder Methacrylsäureestern und/oder Homo- und/oder Copolymeren von Vinylacetat.

Die erfindungsgemäßen Mittel enthalten beschichtete Partikel, die einen mittleren Teilchendurchmesser von 50 bis 500 µm besitzen, bevorzugt von 100 µm bis 250 µm.

Sofern die Hülle nicht zu 100 % aus den genannten Substanzen besteht, kann sie weitere Inhaltsstoffe wie Farb- und Duftstoffe oder Hilfsstoffe enthalten.

Besonders bevorzugte erfindungsgemäße Mittel enthalten in der Hülle der Partikel Weichmacher für eine bessere Elastizität der Hülle. Weichmacher stammen bevorzugt aus der Gruppe Dialkylphthalat, insbesondere Diethylphthalat, Triethylcitrat, Glycerintriacetat und/oder der Polyethylenglycole.

In einer weiteren Ausführungsform enthalten die erfindungsgemäßen Mittel beschichtete Partikel, deren den Partikelkern umgebende Hülle zusätzlich mindestens ein Desintegrationsmittel enthält.

Derartige Desintegrationsmittel werden in der Literatur häufig auch als Zerfallsmittel oder Sprengmittel beschrieben. Derartige Substanzen werden in die Polymerumhüllung eingearbeitet, um deren Zerfallszeiten zu verkürzen. Dieser Zerfall oder diese Sprengung geschieht insbesondere durch eine Volumenvergrößerung infolge von Wasserzutritt (Quellung).

Gemäß Römpp (9. Auflage, Bd. 6, S. 4440) und Voigt "Lehrbuch der pharmazeutischen Technologie" (6. Auflage, 1987, S. 182-184) werden unter solchen Desintegrationsmitteln bzw. Zerfallsbeschleunigern Hilfsstoffe verstanden, die für den raschen Zerfall von Formkörpern in Wasser oder Magensaft und für die Freisetzung der eingeschlossenen Pharmaka in resorbierbarer Form sorgen.

Als bevorzugte Desintegrationsmittel werden im Rahmen der vorliegenden Erfindung Desintegrationsmittel auf Cellulosebasis eingesetzt. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht als einzige Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist. Geeignete Carboxymethylcellulosederivate werden beispielsweise unter dem Handelsnamen Tylopur^{®} von der Firma Clariant oder Ac-Di-Sol^{®} der Firma FMC vertrieben.

Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind. Geeignete mikrokristalline Cellulose ist beispielsweise unter den Handelsnamen Emcocel^{®} von der Firma JRS Pharma oder Avicel^{®} von der Firma FMC kommerziell erhältlich.

Weiterhin kann bevorzugt auch Stärke als Desintegrationsmittel im Rahmen der vorliegenden Erfindung eingesetzt werden. Die erfindungsgemäß einsetzbare Stärke wird üblicherweise aus pflanzlichen Rohstoffen, wie Reis, Soja, Kartoffeln oder Mais gewonnen. Stärke kann unmodifiziert oder analog zur Cellulose als modifizierte Stärke eingesetzt werden. Besonders bevorzugte Stärke-Modifikationen liefern dabei Veresterungs- und Veretherungsreaktionen, insbesondere die erhaltenen Ether aus Reaktionen mit Hydroxycarbonsäuren. Eine erfindungsgemäß besonders geeignete Stärkemodifikation ist die Mischung aus Natriumcarboxymethylstärke und Natriumgylykolstärke, die unter dem Handelsnamen Explotab^{®} durch die Firma JRS Pharma vertrieben wird.

Erfindungsgemäß besonders bevorzugt sind Desintegrationsmittel auf Maisstärke-Basis. Geeignete, modifizierte Maisstärken sind beispielsweise unter den Handelsnamen Glycolys^{®} von der Firma Roquette oder Starch 1500^{®} von der Firma Colorcon erhältlich.

Schließlich stellen Desintegrationsmittel aus vernetztem, wasserunlöslichen Polyvinylpyrrolidinon (PVP) eine weitere Klasse erfindungsgemäß besonders geeigneter Desintegrationsmittel dar. Die vorteilhafte Vernetzung dieser PVP-Modifikation beruht dabei vornehmlich auf Verwicklungen und Verschlingungen der einzelnen Polymerstränge ineinander. Ein erfindungsgemäß besonders bevorzugtes Desintegrationsmittel auf PVP-Basis wird unter dem Handelsnamen Kollidon^{®} CL durch die Firma BASF vertrieben.

Die erfindungsgemäßen, den Partikelkern umgebenden Hüllen enthalten die Zerfallshilfsmittel insbesondere in Mengen von 0,5 bis 20 Gew.-%, bevorzugt von 1 bis 10 Gew.-%, jeweils bezogen auch das Gesamtgewicht der getrockneten Hülle.

In einer weiteren Ausführungsform enthalten die erfindungsgemäßen Mittel beschichtete Partikel, deren den Partikelkern umgebende Hülle zusätzlich mindestens ein Trennmittel enthält.

Als Trennmittel haben sich insbesondere Talkum, amorphes Siliciumdioxid, welches beispielsweise unter dem Handelsnmaen Syloid^{®} 244FP von der Firma Grace GmbH vertrieben wird, Glycerinmonostearat, Magnesiumstearat und Silikate wie Aerosil bewährt. Bevorzugte erfindungsgemäße Mittel enthalten in der Hülle als Trennmittel Talkum.

In einer weiteren Ausführungsform enthalten die erfindungsmäßen Mittel beschichtete Partikel, deren den Partikelkern umgebende Hülle zusätzlich mindestens einen Porenbildner enthält.

Porenbildner werden in die Beschichtung mit eingearbeitet und bewirken, dass sich in der Oberfläche der Beschichtung Poren bilden: Dadurch kommt es zu einer Steigerung der Diffusionsrate in die Polymerhülle für hydrophile Substanzen, insbesondere Wasser.

Erfindungsgemäß eignen sich als Porenbildner besonders Polyvinylpyrrolidinon, Zucker und Zuckeralkohole, wie Lactose, Saccharose, Sorbit und Mannit, Polyethylenglykole mit weniger als 600 Ethylenoxideinheiten, sowie Cellulosederivate, wie Hydroxypropylcellulose, Hydroxypropylmethylcellulose, Methylcellulose und deren Mischungen.

Erfindungsgemäß besonders bevorzugte Porenbildner sind Polyvinylpyrrolidinone (PVP), die beispielsweise unter dem Handelsnamen Kollidon® von der Firma BASF vertrieben werden.

Erfindungsgemäß ist der Gewichtsanteil der Porenbildner in getrockneten Hülle zwischen 0,5 und 20 Gew.-%, insbesondere zwischen 1 und 10 Gew.-%, jeweils bezogen auf das Gesamtgewicht der Hülle

Das Alkalisierungsmittel dient zur Einstellung eines alkalischen pH-Wertes der Anwendungsmischung.

Die erfindungsgemäßen Mittel enthalten Partikel mit einem Partikelkern aus festen Alkalisierungsmitteln, die ausgewählt sind aus Alkalimetall-carbonaten, - phosphaten und/oder -silikaten. Erfindungsgemäß besonders bevorzugte Alkalisierungsmittel sind Alkalimetall-silikate, insbesondere -metasilikate.

In einer weiteren bevorzugten Ausführungsform enthalten die erfindungsgemäßen Mittel mindestens zwei unterschiedliche Alkalisierungsmittel. Dabei können Mischungen beispielsweise aus einem Metasilikat und einem Hydroxycarbonat bevorzugt sein.

Die erfindungsgemäßen Mittel enthalten Alkalisierungsmittel (berechnet als unbeschichtetes Alkalisierungsmittel) bevorzugt in Mengen von 1-50 Gew.-%, insbesondere 15-35 Gew.-%, jeweils bezogen auf das gesamte Mittel.

Die erfindungsgemäßen Blondiermittel enthalten vorzugsweise zusätzlich eine Peroxoverbindung. Die Auswahl dieser Peroxoverbindung unterliegt prinzipiell keinen Beschränkungen. Bevorzugte Peroxoverbindungen sind Wasserstoffperoxid (H₂O₂), beispielsweise in Form einer wässrigen Lösung oder in Form eines H₂O₂-Adduktes an feste Träger, wobei Harnstoffperhydrat oder Natriumcarbonat-Peroxohydrat ("Natriumpercarbonat") eine besondere Bedeutung besitzen. Neben Wasserstoffperoxid oder an seiner Stelle können auch andere Peroxoverbindungen in den erfindungsgemäßen Mitteln enthalten sein.

Übliche, dem Fachmann bekannte Peroxoverbindungen sind beispielsweise Ammoniumperoxodisulfat, Kaliumperoxodisulfat, Natriumperoxodisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxodisulfate, insbesondere Kombinationen aus mindestens zwei Peroxodisulfaten.

Bevorzugte erfindungsgemäße Mittel sind daher dadurch gekennzeichnet, dass sie zusätzlich eine feste Peroxoverbindung enthalten, die vorzugsweise ausgewählt ist aus Wasserstoffperoxid-Anlagerungsverbindungen an feste Träger, Ammonium- und Alkalimetall-persulfaten und -peroxodisulfaten, wobei besonders bevorzugte erfindungsgemäße Mittel mindestens 2 verschiedene Peroxodisulfate enthalten.

Die Peroxoverbindungen sind in den erfindungsgemäßen Blondier- bzw. Aufhell- und Färbemitteln bevorzugt in Mengen von 2-80 Gew.-%, insbesondere in Mengen von 5-50 Gew.-% enthalten.

Weiterhin hat es sich als vorteilhaft erwiesen, wenn die erfindungsgemäßen Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

### Bevorzugte nichtionogene grenzflächenaktive Stoffe sind

- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal^{®}B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon^{®}LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform^{®}TGI (Henkel)) und Poly(2)glycerinpolyhydroxystearat (Handelsprodukt: Dehymuls^{®}PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-%, vor.

Als nichtionische Tenside eignen sich insbesondere C8-C22-Alkylmono- und oligoglycoside und deren ethoxylierte Analoga. Insbesondere die nichtethoxylierten Verbindungen haben sich als besonders geeignet erwiesen.

Beispiele für die in den erfindungsgemäßen Blondiermitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, so genannte "Esterquats", wie das unter der Bezeichnung Dehyquart^{®}F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Blondiermittel weitere Wirk-, Hilfs- und Zusatzstoffe, wie beispielsweise
- nichtionische Polymere wie beispielsweise Vinylpyrrolidinon/Vinylacrylat-Copolymere, Polyvinylpyrrolidinon und Vinylpyrrolidinon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammonium-chlorid-Copolymere, mit Diethylsulfat quaternierte Dimethylamino-ethylmethacrylat-Vinylpyrrolidon-Copolymere, Vinylpyrrolidinon-Imidazolinium-methochlorid-Copolymere und quaternierter Polyvinylalkohol,
- zwitterionische und amphotere Polymere wie beispielsweise Acrylamidopropyl-tri-methylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose,Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- Wirkstoffe wie Panthenol, Pantothensäure, Allantoin, Pyrrolidoncarbonsäuren und deren Salze,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B3, B5, B6, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine, Fettalkohole und Fettsäureester,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N2O, Dimethylether, CO2 und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein weiterer Gegenstand der vorliegenden Erfindung ist ein Verfahren zur Herstellung von Mitteln zum Aufhellen von Keratinfasern, gekennzeichnet durch die Schritte
a) Beschichten mindestens eines Partikelkerns, enthaltend mindestens ein festes Alkalisierungsmittel ausgewählt aus Alkalimetall-carbonaten, -silikaten und -phosphaten mit einem Beschichtungsmittel unter
   Ausbildung einer Hülle, die zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus
   i. Homo- und/oder Copolymeren von Methacrylsäure und/oder
      Methacrylsäureestern und/oder
   ii. Homo- und/oder Copolymeren von Vinylacetat
      besteht.
b) Abmischen des/der beschichteten Teilchen mit weiteren Inhaltstoffen von Blondier- und/Färbemitteln für keratinische Fasern.

Bezüglich bevorzugter Ausführungsformen des erfindungsgemäßen Verfahrens gilt hinsichtlich der Stoffauswahl mutatis mutandis das zu den erfindungsgemäßen Mitteln Gesagte.

In weiter bevorzugten erfindungsgemäßen Verfahren enthalten die beschichteten Partikel (inklusive Beschichtung), bezogen auf ihr Gewicht, 10 bis 70 Gew.-% Beschichtungsmaterial.

Im ersten Schritt des erfindungsgemäßen Verfahrens wird mindestens ein partikelförmiger Inhaltsstoff mit mindestens einem der vorstehend genannten Stoffe beschichtet. Dieser Schritt lässt sich in den unterschiedlichsten Apparaturen problemlos durchführen.

Alternativ zum Einsatz eines Mischergranulators kann auch eine Wirbelschichtapparatur zur Ausbildung des Feststoffcoatings genutzt werden. Erfindungsgemäße Verfahren, bei denen die Beschichtung der zu beschichtenden Partikel in einer Wirbelschichtapparatur durchgeführt wird, sind bevorzugt.

Auch hier kann gleichzeitig Flüssigkeit auf die Körner aufgebracht werden. Die Beschichtung kann dabei gleichzeitig mit der Trocknung erfolgen (beispielsweise in einer Wirbelschichtapparatur, in der die Granulate mit einer Lösung oder Dispersion mindestens eines der oben genannten Stoffe beaufschlagt und gleichzeitig getrocknet werden), es ist aber auch möglich und bevorzugt, die Trocknung nach der Beschichtung, also zeitlich anschließend an diese, durchzuführen.

Es kann eine dichte Hülle erzeugt werden, indem eine Lösung oder Dispersion des/der oben genannten Stoffe(s) auf die zu beschichtenden Partikel aufgebracht wird. Diese Lösung bzw. Dispersion kann auch weitere filmbildende Substanzen enthalten. Ein erfindungsgemäßes Verfahren, bei dem die zu beschichtenden Partikelkerne mit einer Lösung oder Dispersion von
i. Homo- und/oder Copolymeren von Methacrylsäure und/oder Methacrylsäureestern und/oder
ii. Homo- und/oder Copolymeren von Vinylacetat
besprüht und getrocknet wird/werden, ist demnach eine weitere bevorzugte Ausführungsform der vorliegenden Erfindung.

Alternative Beschichtungsverfahren, die sich zur Herstellung erfindungsgemäßer Zusammensetzungen eignen, sind das Strahlschichtcoating sowie Schmelzextrusionsverfahren.

## Patentansprüche

1. Mittel zum Aufhellen von Keratinfasern, insbesondere menschlichen Haaren, enthaltend mindestens einen partikelförmigen Bestandteil, der einen Partikelkern, enthaltend mindestens ein festes Alkalisierungsmittel ausgewählt aus Alkalimetall-carbonaten, -silikaten und -phosphasen, umfasst und eine diesen Kern umgebende Hülle aufweist, **dadurch gekennzeichnet, dass** die Hülle zu mindestens 50 Gew.% ihres Gewichts aus
a) Homo- und/oder Copolymeren von Methacrylsäure und/oder
Methacrylsäureestern (Methacrylaten) und/oder
b) Homo- und/oder Copolymeren von Vinylacetat
besteht.

2. Mittel nach Anspruch 1, **dadurch gekennzeichnet, dass** die Hülle vorzugsweise zu mindestens 70 Gew.-%, weiter bevorzugt zu mindestens 90 Gew.-% und insbesondere zu 100 Gew.-% ihres Gewichts aus
a) Homo- und/oder Copolymeren von Methacrylsäure und/oder
Methacrylsäureestern und/oder
b) Homo- und/oder Copolymeren von Vinylacetat
besteht.

3. Mittel nach einem der Ansprüche 1 oder 2, **dadurch gekennzeichnet, dass** die Hülle Polyvinylacetat enthält.

4. Mittel nach einem der Ansprüche 1 oder 2, **dadurch gekennzeichnet, dass** die Hülle mindestens ein Copolymer aus Methacrylsäure, Methylacrylat und Methylmethacrylat enthält.

5. Mittel nach einem der Ansprüche 1 oder 2, **dadurch gekennzeichnet, dass** die Hülle mindestens ein Copolymer aus Methacrylsäure und Ethylacrylat enthält.

6. Mittel nach einem der Ansprüche 1 oder 2, **dadurch gekennzeichnet, dass** die Hülle mindestens ein Copolymer aus (2-Trimethylammonioethyl) methacrylat chlorid und mindestens einem zusätzlichen Monomer enthält, welches ausgewählt wird aus der Gruppe, die gebildet wird aus Ethylmethacrylat, Methylmethacrylat, Ethylacrylat und Methylacrylat.

7. Mittel nach einem der Ansprüche 1 oder 2, **dadurch gekennzeichnet, dass** die Hülle mindestens ein Copolymer aus Methylmethacrylat und Ethylacrylat enthält.

8. Mittel nach einem der Ansprüche 1 bis 7, **dadurch gekennzeichnet, dass** die den Partikelkern umgebende Hülle zusätzlich mindestens ein Desintegrationsmittel enthält.

9. Mittel nach einem der Ansprüche 1 bis 8, **dadurch gekennzeichnet, dass** die den Partikelkern umgebende Hülle zusätzlich mindestens ein Trennmittel enthält.

10. Mittel nach einem der Ansprüche 1 bis 9, **dadurch gekennzeichnet, dass** die den Partikelkern umgebende Hülle zusätzlich mindestens einen Porenbildner enthält.

11. Mittel nach einem der Ansprüche 1 bis 10, **dadurch gekennzeichnet, dass** es als Partikelkern mindestens ein festes Alkalisierungsmittel enthält, das ausgewählt ist aus Alkalimetallsilikaten.

12. Mittel nach einem der Ansprüche 1 bis 11, **dadurch gekennzeichnet, dass** es als Partikelkern mindestens eine Peroxoverbindung enthält.

13. Verfahren zur Herstellung von Mitteln zum Aufhellen von Keratinfasern, **gekennzeichnet durch** die Schritte
a) Beschichten mindestens eines Partikelkerns, enthaltend mindestens ein festes Alkalisierungsmittel ausgewählt aus Alkalimetall-carbonaten, -silikaten und - phosphaten, mit einem Beschichtungsmittel unter Ausbildung einer Hülle, die zu mindestens 50 Gew.-% ihres Gewichts aus
i. Homo- und/oder Copolymeren von Methacrylsäure und/oder
Methacrylsäureestern und/oder
ii. Homo- und/oder Copolymeren von Vinylacetat
besteht.
b) Abmischen des/der beschichteten Teilchen mit weiteren Inhaltstoffen von Blondiermitteln für keratinische Fasern.

14. Verfahren nach Anspruch 13, **dadurch gekennzeichnet, dass** die zu beschichtenden Partikelkerne in einer Wirbelschichtapparatur mit einer Lösung oder Dispersion von
i. Homo- und/oder Copolymeren von Methacrylsäure und/oder
Methacrylsäureestern und/oder
ii. Homo- und/oder Copolymeren von Vinylacetat
besprüht und getrocknet werden.

## Claims

1. An agent for lightening keratin fibers, in particular human hair, containing at least one particulate component which comprises a particle core, which contains at least one solid alkalizing agent selected from alkali metal carbonates, silicates and phosphates, and has a shell surrounding said core, **characterized in that** at least 50 wt.% of the weight of the shell consists of
a) homopolymers and/or copolymers of methacrylic acid and/or
methacrylic acid esters (methacrylates) and/or
b) homopolymers and/or copolymers of vinyl acetate.

2. The agent according to claim 1, **characterized in that** preferably at least 70 wt.%, more preferably at least 90 wt.% and in particular 100 wt.% of the weight of the shell consists of
a) homopolymers and/or copolymers of methacrylic acid and/or
methacrylic acid esters and/or
b) homopolymers and/or copolymers of vinyl acetate.

3. The agent according to either claim 1 or claim 2, **characterized in that** the shell contains polyvinyl acetate.

4. The agent according to either claim 1 or claim 2, **characterized in that** the shell contains at least one copolymer of methacrylic acid, methacrylate and methyl methacrylate.

5. The agent according to either claim 1 or claim 2, **characterized in that** the shell contains at least one copolymer of methacrylic acid and ethyl acrylate.

6. The agent according to either claim 1 or claim 2, **characterized in that** the shell contains at least one copolymer of (2-trimethylammonio)ethyl methacrylate chloride and at least one additional monomer which is selected from the group formed by ethyl methacrylate, methyl methacrylate, ethyl acrylate and methyl acrylate.

7. The agent according to either claim 1 or claim 2, **characterized in that** the shell contains at least one copolymer of methyl methacrylate and ethyl acrylate.

8. The agent according to one of claims 1 to 7, **characterized in that** the shell surrounding the particle core additionally contains at least one disintegration agent.

9. The agent according to one of claims 1 to 8, **characterized in that** the shell surrounding the particle core additionally contains at least one release agent.

10. The agent according to one of claims 1 to 9, **characterized in that** the shell surrounding the particle core additionally contains at least one pore-forming material.

11. The agent according to one of claims 1 to 10, **characterized in that** it contains at least one solid alkalizing agent as the particle core, which agent is selected from alkali metal silicates.

12. The agent according to one of claims 1 to 11, **characterized in that** it contains at least one peroxo compound as the particle core.

13. A method for producing agents for lightening keratin fibers, **characterized by** the steps of
a) coating at least one particle core, which contains at least one solid alkalizing agent selected from alkali metal carbonates, silicates and phosphates, with a coating agent so as to form a shell, at least 50 wt.% of the weight of which consists of
i. homopolymers and/or copolymers of methacrylic acid and/or
methacrylic acid esters and/or
ii. homopolymers and/or copolymers of vinyl acetate,
b) mixing the coated particle(s) with additional ingredients of blonding agents for keratinic fibers.

14. The method according to claim 13, **characterized in that** the particle cores to be coated are sprayed with a solution or dispersion of
i. homopolymers and/or copolymers of methacrylic acid and/or
methacrylic acid esters and/or
ii. homopolymers and/or copolymers of vinyl acetate
in a fluidized bed apparatus, and are dried.

## Revendications

1. Agent d'éclaircissement de fibres de kératine, en particulier de cheveux humains, contenant au moins un composant particulaire qui comprend un noyau de particules, contenant au moins un agent d'alcalinisation solide choisi parmi les carbonates, les silicates et les phosphates de métaux alcalins, et une enveloppe entourant ce noyau, **caractérisé en ce que** l'enveloppe est constituée à au moins 50% de son poids
a) d'homopolymères et/ou copolymères de l'acide méthacrylique et/ou d'esters d'acide méthacrylique (méthacrylates) et/ou
b) d'homopolymères et/ou copolymères d'acétate de vinyle.

2. Agent selon la revendication 1, **caractérisé en ce que** l'enveloppe est constituée de préférence à au moins 70%, plus préférablement à au moins 90% et en particulier à 100%, de son poids
a) d'homopolymères et/ou de copolymères de l'acide méthacrylique et/ou d'esters d'acide méthacrylique et/ou
b) d'homopolymères et/ou copolymères d'acétate de vinyle.

3. Agent selon l'une des revendications 1 ou 2, **caractérisé en ce que** l'enveloppe contient de l'acétate de polyvinyle.

4. Agent selon l'une des revendications 1 ou 2, **caractérisé en ce que** l'enveloppe contient au moins un copolymère d'acide méthacrylique, d'acrylate de méthyle et de méthacrylate de méthyle.

5. Agent selon l'une des revendications 1 ou 2, **caractérisé en ce que** l'enveloppe contient au moins un copolymère d'acide méthacrylique et d'acrylate d'éthyle.

6. Agent selon l'une des revendications 1 ou 2, **caractérisé en ce que** l'enveloppe contient au moins un copolymère de chlorure de (2-triméthylammonioéthyl)-méthacrylate et au moins un monomère supplémentaire qui est choisi dans le groupe qui est formé par le méthacrylate d'éthyle, le méthacrylate de méthyle, l'acrylate d'éthyle et l'acrylate de méthyle.

7. Agent selon l'une des revendications 1 ou 2, **caractérisé en ce que** l'enveloppe contient au moins un copolymère de méthacrylate de méthyle et d'acrylate d'éthyle.

8. Agent selon l'une des revendications 1 à 7, **caractérisé en ce que** l'enveloppe entourant le noyau de particules contient en outre au moins un agent de désintégration.

9. Agent selon l'une des revendications 1 à 8, **caractérisé en ce que** l'enveloppe entourant le noyau de particules contient en plus au moins un agent de démoulage.

10. Agent selon l'une des revendications 1 à 9, **caractérisé en ce que** l'enveloppe entourant le noyau de particules comprend en outre au moins un agent porogène.

11. Agent selon l'une des revendications 1 à 10, **caractérisé en ce qu'**il contient comme noyau de particules au moins un agent d'alcalinisation solide qui est choisi parmi les silicates de métaux alcalins.

12. Agent selon l'une des revendications 1 à 11, **caractérisé en ce qu'**il contient comme noyau de particules au moins un composé peroxo.

13. Procédé de production d'agents d'éclaircissement de fibres de kératine, **caractérisé par** les étapes consistant à
a) revêtir au moins un noyau de particules contenant au moins un agent d'alcalinisation solide choisi parmi les carbonates, les silicates et les phosphates de métaux alcalins, avec un agent de revêtement pour former une enveloppe qui est constituée à au moins 50% de son poids
i. d'homopolymères et/ou de copolymères de l'acide méthacrylique et/ou d'esters d'acide méthacrylique et/ou
ii. d'homopolymères et/ou de copolymères d'acétate de vinyle.
b) mélanger le ou les particules revêtues avec d'autres ingrédients d'agents de blanchiment de fibres de kératine.

14. Procédé selon la revendication 13, **caractérisé en ce que** les noyaux de particules à revêtir sont aspergés et séchés dans un appareil à lit fluidisé avec une solution ou une dispersion
i. d'homopolymères et/ou de copolymères de l'acide méthacrylique et/ou d'esters d'acide méthacrylique et/ou
ii. d'homopolymères et/ou de copolymères d'acétate de vinyle.
